Europäisches Patentamt

(19) European Patent Office

Office européen des brevets

(11) Numéro de publication : **0 190 982**
**B1**

(12) **FASCICULE DE BREVET EUROPÉEN**

(45) Date de publication du fascicule du brevet :
20.09.89

(51) Int. Cl.⁴ : **A 61 F 2/46**

(21) Numéro de dépôt : 86420037.3

(22) Date de dépôt : 06.02.86

(54) **Dispositif d'assemblage entre un implant osseux et l'outil destiné à sa mise en place.**

(30) Priorité : 07.02.85 FR 8501915

(43) Date de publication de la demande :
13.08.86 Bulletin 86/33

(45) Mention de la délivrance du brevet :
20.09.89 Bulletin 89/38

(84) Etats contractants désignés :
CH DE FR IT LI

(56) Documents cités :
CH—A— 619 855
DE—A— 2 213 552
FR—A— 2 242 068
FR—A— 2 345 610
FR—A— 2 395 011

(73) Titulaire : **Etablissement TORNIER, Société dite**
**Chemin Doyen Gosse B.P. 11**
**F-38330 Saint Ismier (FR)**

(72) Inventeur : **Tornier, Alain**
**Le Brucey**
**F-38190 Crolles (FR)**

(74) Mandataire : **Karmin, Roger et al**
**Cabinet MONNIER 150, cours Lafayette**
**F-69003 Lyon (FR)**

Jouve, 18, rue St-Denis, 75001 Paris, France

EP 0 190 982 B1

## Description

On sait que les implants tels que les cotyles artificiels pour tête de fémur, prévus métalliques ou en plastique peuvent être pourvus sur leur périphérie d'un ou plusieurs filets de forme cylindrique tronconique ou sphérique.

Il existe actuellement trois systèmes de mise en place :

tout d'abord on peut pratiquer un taraudage préalable de l'os iliaque, lorsqu'il s'agit de remplacer la cavité cotyloïde, puis la prothèse est mise en place par vissage ;

dans certains cas le filet extérieur de la prothèse est réalisé comme un taraud de manière à permettre la mise en place directe de la prothèse dans l'os ;

si la prothèse comporte un élément métallique pourvu d'une cupule en matière plastique, cette dernière est mise en place une fois l'élément métallique associé a l'os.

Les perfectionnements qui font l'objet de la présente invention ont pour objet de faciliter la mise en place des cotyles artificiels.

La majeure partie des systèmes de mise en place actuellement connus comportent une clef ou manche d'implantation libre par rapport au cotyle, ce qui interdit le contrôle de la direction de l'un de ce dernier, lors de l'implantation.

Il existe aussi, par exemple dans le brevet FR-A-2395011, des systèmes de fixation du cotyle artificiel comportant des vis d'assemblage à la clef. Mais compte-tenu des conditions opératoires, ce système est d'utilisation mal aisée et la séparation des pièces est difficile car elle doit s'effectuer directement dans le foyer opératoire. De plus, le démontage des vis d'assemblage est long, ce qui entraîne une perte de temps néfaste pour le malade et la bonne marche de l'opération.

Suivant l'invention, le cotyle artificiel comporte une collerette annulaire périphérique dans laquelle sont ménagées des encoches comportant au moins une paroi en contre-dépouille, tandis que la clef d'implantation est établie sous la forme d'un manche pourvu de dents en forme de coin qu'on peut engager dans les encoches de manière que la face oblique des coins vienne en appui contre la paroi en contre-dépouille des encoches grâce à des cales coulissant axialement et qui viennent s'engager dans lesdites encoches. Le manche peut comprendre une poignée constituée par une barre oblique qui est associée à un système connu par exemple du type clef à cliquet. Ainsi, le manche permet de maintenir parfaitement l'orientation désirée de l'axe géométrique du cotyle, tandis que la barre est utilisée pour faire tourner la prothèse qui est mise en place par taraudage direct, ce qui évite le taraudage préalable et donc la difficulté de retrouver le filet déjà usiné dans le cotyle lors de la mise de la prothèse, Si cette dernière est composite, c'est-à-dire qu'elle comporte un élément extérieur métallique et une cupule intérieure en matière plastique, on peut mettre une telle prothèse composite en place directement, l'assemblage de ses deux éléments ayant été réalisé en usine et non pendant l'opération, ce qui diminue encore la longueur de celle-ci.

Le dessin annexé, donné à titre d'exemple, permettra de mieux comprendre l'invention, les caractéristiques qu'elle présente et les avantages qu'elle est susceptible de procurer :

Fig. 1 est une vue en perspective d'un dispositif d'assemblage établi conformément à l'invention.

Fig. 2 à 5 montrent la manière dont les deux parties du dispositif sont verrouillées l'une par rapport à l'autre.

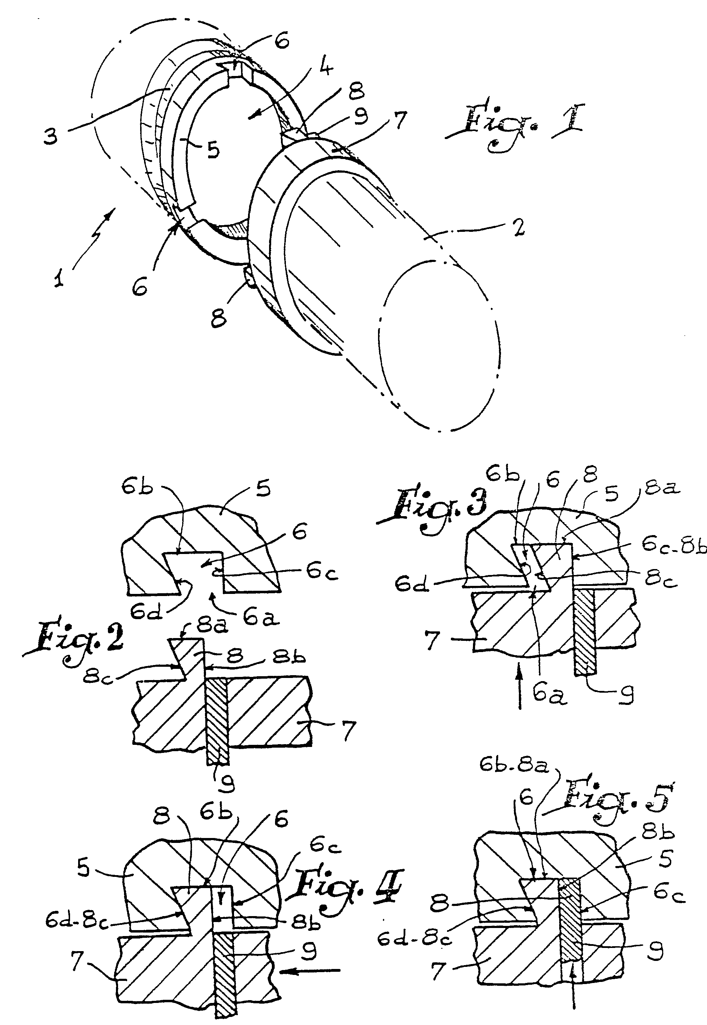

Le dispositif d'assemblage suivant l'invention est destiné à solidariser un implant 1, par exemple un cotyle artificiel et un outil de mise en place ou manche 2 illustré en traits discontinus en fig. 1.

La périphérie 3 du cotyle 1 affecte la forme d'un taraud usuel qu'on a simplement représenté par des traits discontinus pour ne pas surcharger la figure. Bien entendu, ce taraud peut être à un ou plusieurs filets. Autour de sa cavité centrale 4, le cotyle comporte une collerette annulaire 5 pourvue de trois encoches 6. Comme illustré, chaque encoche présente la forme d'un trapèze rectangle dont la petite base correspond à l'ouverture 6a de l'encoche et la grande base 6b au fond de celle-ci. Ces deux bases sont reliées par une paroi normale 6c et par une paroi oblique en contre-dépouille 6d.

Le manche 2 comporte à l'une de ses extrémités une douille 7 comportant des dents axiales 8 en forme de coin en nombre identique à celui des encoches 6 et réparties de la même manière, la bague 7 présente le même diamètre extérieur que la collerette 5 de telle façon que les dents 8 puissent s'engager librement dans les encoches 6. A cet effet, et comme illustré en fig. 2, chaque dent 8 affecte la forme d'un trapèze rectangle dont les bases sont plus petites que celles des encoches 6. En particulier, la grande base 8a de la dent présente une dimension inférieure à celle de la petite base 6a ou ouverture de l'encoche 6 de manière qu'on puisse librement engager la dent dans celle-ci. La hauteur de chaque dent 8 est légèrement supérieure à la profondeur des encoches.

La face 8b de chaque dent qui se trouve orientée perpendiculairement à sa grande base 8a reçoit à coulissement une cale 9 déplaçable axialement et dont l'épaisseur est telle que lorsque cette cale est placée à côté de la dent 8, ces deux organes constituent un ensemble dont la forme correspond très exactement à celle de l'encoche 6.

Le coulissement des cales 9 s'effectue au moyen d'une commande non représentée accessible à l'extrémité du manche opposée à la douille 7.

La solidarisation du manche 2 et de l'implant 1 s'effectue comme illustré en fig. 3 à 5 : les trois dents 8 sont d'abord engagées dans les trois encoches correspondantes 6 par un mouvement axial du manche par rapport à l'implant, puis l'implant étant fixe, on opère une petite rotation du manche, de manière que les faces obliques 8c des dents 8 dont la pente est la même que celle des parois 6d des encoches, viennent en appui sur ces dernières (fig. 4). Enfin, la dernière opération consiste à déplacer les trois cales 9 de manière simultanée dans le sens axial afin qu'elles pénètrent dans les encoches 6 et maintiennent comprimées les dents par rapport aux encoches afin que la face en biais 8c de chaque dent soit bloquée contre la paroi oblique 6d de l'encoche correspondante, de telle sorte qu'après cette opération, le manche 2 et la prothèse 1 soient fermement assujettis pour ne former en fait qu'une seule et même pièce.

Comme indiqué plus haut, le manche 2 pourrait comprendre une couronne à rochet de manière qu'une bague comportant un cliquet puisse entraîner ce manche en rotation dans un sens et dans l'autre, par exemple au moyen d'une tige oblique solidaire de ladite bague.

Il doit d'ailleurs être entendu que la description qui précède n'a été donnée qu'à titre d'exemple et qu'elle ne limite nullement le domaine de l'invention dont on ne sortirait pas en remplaçant les détails d'exécution décrits par tous autres équivalents.

**Revendications**

1. Dispositif d'assemblage entre un implant destiné à être fixé à un os notamment un cotyle artificiel (1), et un manche (2), caractérisé en ce qu'il comprend d'une part des dents (8) en forme de coin montées sur l'extrémité dudit manche (2), et des cales (9) adjacentes aux coins, montées coulissantes par rapport à ceux-ci et dans la direction de l'axe longitudinal du manche, et d'autre part des encoches (6) avec une face (6d) en contre-dépouille ménagée dans l'implant (1) et dans chacune desquelles se loge une dent (8) et sa cale (9), la face en biais (8c) de chaque coin venant en appui contre la face en contre-dépouille (6d) de l'encoche correspondante quand la cale vient se loger dans l'encoche considérée.

2. Dispositif suivant la revendication 1, caractérisé en ce que la commande permettant le coulissement axial des cales (9) s'effectue à partir de l'extrémité libre du manche opposée à celle qui porte les dents (8) afin que la commande considérée soit située loin de l'implant (1).

3. Dispositif suivant la revendication 1, caractérisé en ce que les encoches (6) sont ménagées dans une collerette annulaire périphérique (5) de l'implant (1).

**Claims**

1. Device for fitting together an implant intended to be fixed to a bone, in particular an artificial acetabulum (1), and a sleeve (2), characterized in that it comprises on the one hand wedge-shaped teeth (8) mounted on the end of the said sleeve (2) and blocks (9) adjacent to the wedges and mounted sliding with respect to the latter in the direction of the longitudinal axis of the sleeve, and on the other hand recesses (6) with one undercut face (6d) made in the implant (1), in each of which there is received a tooth (8) and its block (9), the slanting face (8c) of each wedge coming to bear against the undercut face (6d) of the corresponding recess when the block is received in the said recess.

2. Device according to Claim 1, characterized in that the control allowing axial sliding of the blocks (9) is effected from the free end of the sleeve opposite that carrying the teeth (8), in order that the said control is located remote from the implant (1).

3. Device according to Claim 1, characterized in that the recesses (6) are made in a peripheral annular flange (5) of the implant (1).

**Patentansprüche**

1. Vorrichtung zum Verbinden eines an einem Knochen zu befestigenden Implantats, insbesondere einer künstlichen Gelenkpfanne (1), mit einem Werkzeug für seine Einsetzung (2), dadurch gekennzeichnet, dass sie einerseits die Form von Keilen besitzende Zähne (8), die am Ende des Werkzeugs (2) befestigt sind, und Klemmschieber (9), die zu den Keilen benachbart sind sowie gleitend bezüglich dieser und in Richtung der Längsachse des Werkzeugs angeordnet sind, und anderseits Aussparungen (6) aufweist, welche eine im Implantat (1) ausgenommene, eine Formschräge darstellende Seitenfläche (6d) besitzen und in welchen jeweils ein Zahn (8) und sein Klemmschieber (9) zu liegen kommen, wobei die geneigte Seitenfläche (8c) jedes Keils gegen eine eine Formschräge darstellende Seitenfläche (6d) der entsprechenden Aussparung in Anlage kommt, wenn der klemmschieber in der zugehörigen Aussparung plaziert wird.

2. Vorrichtung nach Anspruch 1, dadurch gekennzeichnet, dass die Betätigung, welche das axiale Gleiten der Klemmschieber (9) ermöglicht, von dem freien Ende des Werkzeuges erfolgt, das demjenigen gegenüberliegt, welches die Zähne (8) trägt, so dass die Betätigung weit weg vom Implantat (1) erfolgt.

3. Vorrichtung nach Anspruch 1, dadurch gekennzeichnet, dass die Aussparungen (6) in einem umlaufenden Ringkragen (5) des Implantats (1) ausgenommen sind.

Fig. 1

Fig. 2

Fig. 3

Fig. 4

Fig. 5